Europäisches Patentamt

European Patent Office

Office européen des brevets

(19)

(11) Veröffentlichungsnummer: **0 617 982 A1**

## (12) EUROPÄISCHE PATENTANMELDUNG

(21) Anmeldenummer: **94103719.4**

(51) Int. Cl.5: **A61N 2/00**

(22) Anmeldetag: **10.03.94**

(30) Priorität: **11.03.93 DE 4307767**

(43) Veröffentlichungstag der Anmeldung:
**05.10.94 Patentblatt 94/40**

(84) Benannte Vertragsstaaten:
**AT CH DE FR GB IT LI**

(71) Anmelder: **ZENTRALINSTITUT FÜR BIOMEDIZINISCHE TECHNIK UNIVERSITÄT ULM**
**Albert-Einstein-Allee 11**
**D-89081 Ulm (DE)**

(72) Erfinder: **Edrich, Jochen, Prof. Dr. Dr.**
**Albert Schweitzerstrasse 12**
**D-89257 Tiefenbach-Illertissen (DE)**
Erfinder: **Zhang, Tongsheng, Dr.**
**Memmingerstrasse 24,**
**Wiley Kaserne,**
**Appart. 273**
**D-89238 Neu-Ulm (DE)**

(74) Vertreter: **Behrens, Dieter, Dr.-Ing.**
**Wuesthoff & Wuesthoff**
**Patent- und Rechtsanwälte**
**Schweigerstrasse 2**
**D-81541 München (DE)**

(54) **Verfahren und Vorrichtung zur neuromagnetischen Stimulation.**

(57) Bei einem Verfahren und einer Vorrichtung zur neuromagnetischen Stimulation von Nervensträngen werden gleichzeitig ein magnetisches Feld mit einer magnetischen Induktion B und wenigsten ein fokussierter Ultraschallstrahl angelegt. Das magnetische Feld steht dabei orthogonal zur Ultraschallschwingungsrichtung. Es wird ein Fokus von ca. 1 cm Durchmesser erzeugt. So lassen sich die für eine wirksame und gezielte Stimulation erforderlichen relativ starken Felder auf ein einziges Nervenbündel oder einem einzigen zentralen Nervenbereich richten. Es wird verhindert, daß benachbarte Nervenbereiche ungewollt mitstimuliert werden.

FIG. 1

EP 0 617 982 A1

Die Erfindung betrifft ein Verfahren und eine Vorrichtung zur funktionellen, neuromuskulären bzw. neuromagnetischen Stimulation (Stimulation von Nerven durch Anlegen eines Magnetfelds), bei dem das zu stimulierende Nervengewebe mit einer magnetischen Induktion beaufschlagt wird.

Funktionelle, neuromuskuläre (neuromagnetische) Stimulation hat sich in jüngster Zeit weiterentwickelt, besonders in Form der nicht-kontaktierenden, magnetischen Stimulation. Die neuromagnetische Stimulation hat viele Anwendungen in der Forschung. Aber auch klinische Anwendungen ergeben sich, u.a. in der Neurologie, der Rehabilitations-Medizin und der nichtinvasiven funktionellen Stimulation von zentralen und peripheren Nerven, u. a. bei der Diagnose. Repräsentative Veröffentlichungen sind: D. Cohen and N.B. Cuffin, Developing a more focal stimulator, Journal of Clinical Neurophysiology, 1991, S. 102-120; S.N. Erné and J. Edrich, Designing multichannel sensor arrays for biomagnetism, Proc. IEEE-EMBS Conference Paris, CH3207, 1992, S. 1772/1773; B.A. Evans, Magnetic stimulation of the peripheral nervous system, Journal of Clinical Neurophysiology, 1991, S. 77-84; and A.T. Barker, An introduction to the basic principles of magnetic nerve stimulation, Journal of Clinical Neurophysiology, 1991, S. 26-37. Effizienz und Spezifität der magnetischen Stimulation sind sehr eingeschränkt, da eine wichtige, räumliche Fokussierung der dabei benützten magnetischen Felder auf ein Volumen von weniger als 1 cm$^3$ bisher nicht möglich ist. Eine genaue Fokussierung der erforderlichen relativ starken Felder (ca. 1 Tesla) auf ein einziges Nervenbündel oder auf einen einzigen zentralen Nervenbereich, wie z.B. das motorische Areal des Daumens im präzentralen Kortex-Bereich, ist aber notwendig, damit benachbarte Areale, wie zum Beispiel, das weniger als einen Zentimeter entfernte Areal des Zeigefingers, nicht ungewollt mitstimuliert werden.

Der Einsatz von Ultraschall und dafür geeigneter Vorrichtungen ist auch bereits Gegenstand von Veröffentlichungen gewesen: L.A. Frizzell, Threshold dosages by high intensity focused ultrasound, IEEE Trans. Biomed.Eng. 24, 1988, S. 578-581; J.A. Evans and M.B. Tavakoli, Ultrasonic attenuation and velocity in bone, Phys. Med. Biol. Vol. 35, 1990, No.10; and S. Umemura and C.A. Cain, Acoustical evaluation of a prototyp sector-vortex phased-array applicator, IEEE Trans. on Ultrasonics, Ferroelectrics and Frequency Control., Vol. 39, 1992, No. 1, S. 32-38.

Der Erfindung liegt das Problem zugrunde, Verfahren und Vorrichtung zur neuromagnetischen Stimulation zu schaffen, denen die Mängel der bekannten Magnetstimulation nicht anhaften und die eine genauere räumliche Stimulation ermöglichen.

Zur Lösung dieses Problems sieht die Erfindung Verfahren und Vorrichtung vor, wie sie in den Patentansprüchen gekennzeichnet sind, bei denen dem angelegten Magnetfeld eine fokussierte Ultraschallschwingung derart überlagert wird, daß sie orthogonal zum Magnetfeld schwingt und ein subkutaner Fokus von nicht mehr als ca. 1 cm Durchmesser erzeugt wird.

Der Ultraschall wird zweckmäßigerweise gepulst oder reihengepulst und zur Fokussierung amplituden- und phasengesteuert. Bevorzugt ist ein Frequenzbereich von 0,2 bis 3 MHz. Im Frequenzbereich 0,3 bis 0,5 MHz ist die enge subkutane Fokussierung besonders gut realisierbar. Das Magnetfeld mit der Induktion B kann ein Gleichfeld oder ein gepulstes oder sogar reihengepulstes Magnetfeld sein. Magnetfeld und Ultraschall sind im Fokusbereich zweckmäßigerweise bezüglich Phase und Frequenz abzugleichen.

Ein Ausführungsbeispiel der Erfindung ist anhand einer Zeichnung näher erläutert, in der zeigt:

Fig. 1    einen Längsquerschnitt von einer Ultraschallquelle zu einem Nervengewebe,

Fig. 2    die x-y-Querschnittsebene durch den Ultraschallfokus im Gewebe, und

Fig. 3    ein Blockschaltbild einer Vorrichtung zur Ausführung des erfindungsgemäßen Verfahrens.

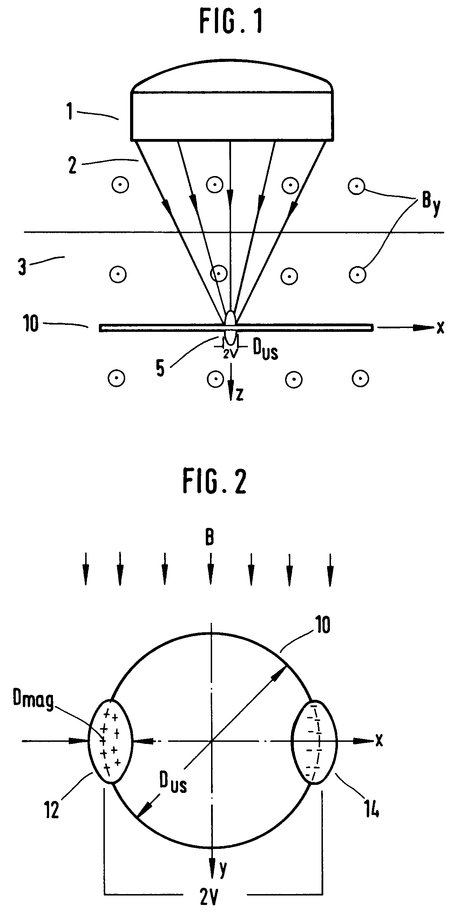

Gemäß Fig. 1 erzeugt eine Ultraschallwellen 2 aussendende fokussierende Ultraschall-Strahlungsquelle 1 innerhalb eines Gewebes 3 einen subkutanen Ultraschallfokus(bereich) 5 von ca. 1 cm Durchmesser senkrecht zu ihrer Hauptstrahlachse z. Dies ist im Frequenzbereich 0,2 bis 3 MHz derzeit gut und im Bereich von Frequenzbereich 0,3 bis 0,5 MHz besonders gut realisierbar. Bei ausreichender Leistungsdichte $P_{us}$ im Bereich des Ultraschallfokus kommt es dadurch zu Vibrationen der Gewebepartikel bei dieser Frequenz. Die im Nervengewebe enthaltenen Ladungsträger und besonders die Elektronen werden dabei mitbewegt, was einem Wechselstrom J entspricht. Wird nun gleichzeitig ein Magnetfeld mit magnetischer Induktion B orthogonal zur Vibration bzw. Stromrichtung x angelegt, so wirkt auf die bewegten Ladungsträger die sogenannte Lorentz-Kraft F orthogonal zu B und J; dabei kann das B-Feld ein Gleichfeld oder ein gepulstes Magnetfeld sein. Die gleichzeitige Einwirkung eines konstantes B-Feldes und eines fokussierten Ultraschalles hat, wie in Fig. 2 gezeigt, im Bereich x>0 eine Anhäufung von negativen Ladungsträgern und im Bereich x<0 eine gleichgroße Anhäufung von positiven Ladungsträgern zur Folge. Die dadurch bedingte Spannung 2V zwischen beiden Ladungsbereichen ist gegeben durch

$$V = B \cdot D_{us} \cdot U_z \qquad (1)$$

worin $D_{us}$ der Durchmesser des Utraschallfokusses 5 in transversaler Richtumg x und $U_z$ die durch den

2

Ultraschall bedingte Ladungsträgergeschwindigkeit sind. Diese Ladungsträgergeschwindigkeit $U_z$ ist mit der Ultraschall-Leistungsdichte $P_{us}$, der spezifischen Gewebedichte rho und der Ausbreitungsgeschwindigkeit von Ultraschall $c_{us}$ im Gewebe verknüpft durch die Beziehung

$$U_t = (2 \cdot P_{us}/rho \cdot c_{us})^{1/2} \qquad (2)$$

Zur Abschätzung der numerischen Verhältnisse diene folgendes Zahlenbeispiel:

rho = 1000 kg/cm$^3$
$c_{us}$ = 1500 m/sec
B = 1 Tesla
$P_{us}$ = 2000 W/cm$^2$
$D_{us}$ = 0,01 m
f = 0,5 MHz

Mittels der beiden obigen Gleichungen errechnet sich daraus eine induzierte Spannung

$$V = B \cdot D_{us} \cdot (2 \cdot P_{us}/rho \cdot c_{su})^{1/2}$$

$$= 1 \text{Tesla} \cdot 0{,}01\text{m} \cdot \left(\frac{2 \cdot 2000 \ W/cm^2}{1000 kg/m^3) \cdot 1500 m/s}\right)^{1/2} = 103 \ mV,$$

welche zur depolarisierenden Nervenstimulation führt. Die Ultraschallfrequenz ist dabei mit 0,4 MHz angesetzt, bei welcher die Wellenlänge l (lambda)

$$l = c_{us}/f = (1500 \ m/sec)/0{,}5 \cdot 10^6 \ Hz = 3 \ mm. \qquad (3)$$

beträgt. Im Idealfeld ist es nun theoretisch möglich, einen minimalen Wert für den Fokusdurchmesser von

$$D_{us} = l/2 = 1{,}5 \ mm \qquad (4)$$

zu erreichen. Der oben angenommene Wert $D_{us}$ = 1 cm kann daher auch praktisch sicher eingehalten werden.

In Fig. 2, die eine x-y-Querschnittsebene 10 des Ultraschallfokus 5 von Fig. 1 auf die der Ultraschall auf die Querschnittsebene 10 einfällt, zeigt, sind die Konzentrationsbereiche 12, 14 derLadungsträger eingezeichnet, welche zur depolarisierenden Stimulation führen. Der Konzentrationsbereich 12 der positiven Ladungsträger und der dazu diametrale Konzentrationsbereich 14 negativer Ladungsträger entsteht durch gleichzeitiges Einwirken des fokussierten Ultraschalls und des Magnetfeldes B. Reihenimpulse können die effektive Pulsbreite der Stimulationsspannung erhöhen und dadurch die Erregungsschwelle um ein Vielfaches erniedrigen. Die zur Depolarisation führende positiv geladene Region in Fig. 2 hat in x-Richtung eine 3 dB-Breite $D_{mag}$ = 0.4$\cdot D_{us}$ = 4 mm. Auch in der Tiefe von vielen cm beschreibt $D_{mag}$ die Fokussiergenauigkeit, welche um ein Vielfaches größer als bei konventioneller Magnetstimulationen ist.

Eine zusätzliche Erhöhung der Fokussiergenauigkeit kann durch Amplituden- und Phasenregelung der als fokussierendes Ultraschall-Array ausgebildeten Ultraschallquelle erzielt werden, die durch Daten aus anatomischen Strukturbildern von CAT, MRI oder diagnostischem Ultraschall gesteuert werden.

Die Formen und die gegenseitigen Zuordnungen der angewandten magnetischen und Ultraschall-Wellen müssen geeignet ausgeführt sein, um ein optimales Stimulationsverhalten zu erzielen. Dies kann von dem induzierten elektrischen Feld $E_x$ abgeleitetwerden, welches aus dem Gradienten der Spannung V der Gleichungen (1) und (2) ergibt:

$$E_x = \frac{\delta V}{\delta x}$$
$$= - B_o \cdot (2P_{us}(x,y,z)/rho \cdot c_{us})^{1/2} \cdot \cos(2\pi ft - 2\pi z/l) \qquad (5)$$

wobei l (lambda) die Wellenlänge des Ultraschalls im Gewebe ist. Man kann nun das magnetische Feld $B_y$ bei der gleichen Frequenz anlegen:

$$B_y = Bo \cdot \cos(2\pi ft - \alpha). \qquad (6)$$

Die Verbindung beider Gleichungen (5) und (6) ergibt:

$E_x = -\frac{\partial V}{\partial x}$

$= -B_o \bullet (2P_{us}(x,y,z)rho \bullet c_{us})^{1/2} \bullet \cos(2\pi ft - 2\pi z/l) \bullet \cos(2\pi ft - \alpha)$     (7)

Das Feld $|E_x|$ erreich für t

$\alpha = 2\pi z_o/l + 2n\pi$, (n = ganze Zahl)     (8)

in der Umgebung des Fokuspunktes (z = $z_o$) sein Maximum

$|E_x|_\alpha = B_o \bullet (2P_{us}(x,y,z)/rho \bullet c_{us})^{1/2} \bullet \cos^2(2\pi ft - 2\pi z/l)$
$= E_{max} \bullet \cos(2\pi ft - 2\pi z/l)$     (9)

Diese Gleichungen zeigen, daß das induzierte elektrische Feld im synchronisierten Fall eine "stehende Welle" mit einem Maximum im Fokus ergibt, das einen dazugehörigen Wert hat, der um einen Faktor zwei größer ist als im nichtsynchronisierten Fall.

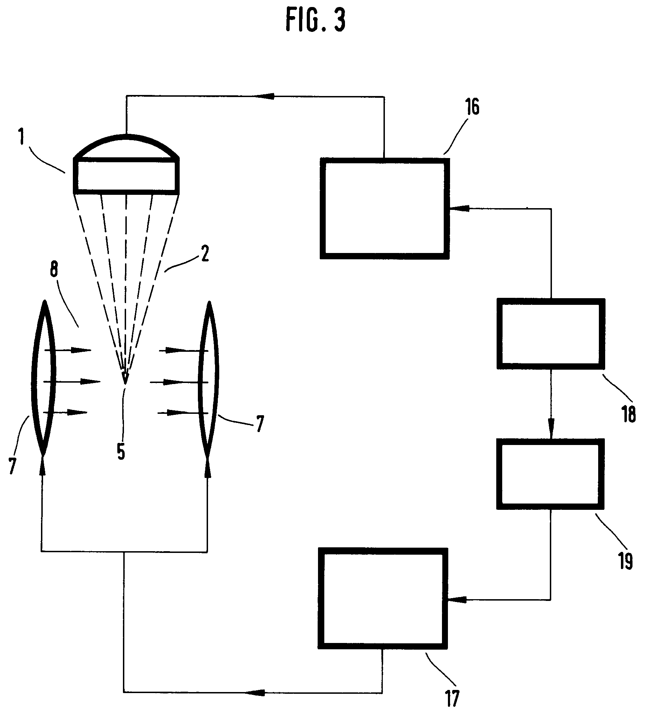

Fig. 3 zeigt ein Blockschaltbild einer praktischen Ausführung, welche die Ultraschallquelle 1 einschließt, welche Ultraschallwellen 2 auf den Fokus 5 fokussiert. Spulen 7 erzeugen die magnetischen Felder 8. Die Ultraschallquelle 1 und die Spulen 7 werden durch die Verstärker 16 und 17 gespeist. Ein Synchronisationsschaltkreis 18 und ein Verzögerungskreis 19 erzeugen die Frequenz- und Phasenregelung. Mittels des Verzögerungskreises 19 kann der Stimulationspunkt verändert werden, was zu einer hochgradigen Selektivität bei dieser Art der Stimulation führt. Anstatt der sinusförmigen Magnet- und Ultraschall-Felder kann man auch gepulste oder seriengepulste Felder verwenden. Ein maximales E-Feld wird wiederum durch Synchronisation, d.h. durch Phasen- und Frequenz-Gleichheit für diese zwei Größen erzielt.

Für die Anwendug im klinisch wichtigen zerebralen Bereich existieren hauptsächlich die folgenden zwei Probleme:

(1) die kraniale Knochenstruktur produziert Dämpfungen von 10 bis 20 dB;

(2) durch die Reflektionen können unerwünschte Sekundärfokusse im intrakraniellen Bereich entstehen.

Beide Probleme können mittels mehrerer fokussierender Ultraschallsysteme bzw. -sender gelöst werden, die in helmartiger Form den Schädel umschließen. Dadurch wird die Leistungsdichte im Fokusbereich um ein Vielfaches erhöht, ohne überhöhte zusätzliche Belastungen in präfokalen Regionen zu bewirken. Rückkopplung über CAT, MRI oder diagnostischer Ultraschall und die oben erwähnten Amplituden- und Phasenregler können wiederum zur weiteren Reduzierung von Sekundärfokussen eingesetzt werden.

Da die Fokussierung im wesentlichen durch den Ultraschall bestimmt wird, können die Magnetspulen relativ groß ausgeführt werden, um die Erzeugung von großen Magnetfeldern zu erleichtern, ohne dabei einen so gravierenden Fokussierverlust zu erleiden, wie er für herkömmliche Magnetstimulation üblich ist.

Zur weiteren Erniederung von überhöhten präfokalen Feldern können Mehrfachspulensysteme ähnlich den Vielkanalsystemen für Magnetoenzephalographie und Magnetokardiographie zur Anwendung kommen.

Die Wahrscheinlichkeit von instabiler Kavitation durch Ultraschall steigt mit wachsender Ultraschall-Leistungsdichte. Für die meisten Körperregionen liegt diese Schwelle oberhalb von 6000 W/cm². Die hier beschriebene Methode liegt mit maximal 2000 W/cm² in sicherem Abstand darunter.

Eine Überwärmung durch fokussierten Ultraschall wird in der onkologischen Hyperthermie angewandt. Die dabei erfolgende zeitliche Temperaturerhöhungsrate dT/dt kann man bei Vernachlässigung von abgeleiteter Wärme berechnen durch die Beziehung

$dT/dt = 2\alpha \bullet P_{us}/rho \bullet c_m$     (10)

Nimmt man typische Gewebedaten (Utraschall-Absorptiortskoeffizient $\alpha$ = 0.015/cm, spezifische Wärme $c_m$ = 4,2 Joule/(gK)) bei einer Ultraschallfrequenz von 0,5 MHz an, so erhält man für eine Ultraschallpulsbreite dt = 1 ms eine Temperaturerhöhung von 0,014 K, also eine vernachlässigbare Erwärmung.

**Patentansprüche**

1. Verfahren zur neuromagnetischen Stimulation, bei dem das zu stimulierende Nervengewebe mittels eines Magnetfelds mit einer magnetischen Induktion B beaufschlagt wird,

dadurch gekennzeichnet,
daß dem angelegten Magnetfeld eine fokussierte Ultraschallschwingung derart überlagert wird, daß sie orthogonal zum Magnetfeld schwingt, und daß ein subkutaner Fokus von nicht mehr als ca. 1 cm Durchmesser erzeugt wird.

2. Verfahren nach Anspruch 1, dadurch gekennzeichnet, daß die Ultraschallfreqenz im Bereich von 0,2 bis 3 MHz, vorzugsweise im Bereich von 0,3 bis 0,5 MHz, liegt.

3. Verfahren nach Anspruch 1 oder 2, dadurch gekennzeichnet, daß der Ultraschall gepulst, insbesondere reihengepulst ist.

4. Verfahren nach Anspruch 1, dadurch gekennzeichnet, daß der Ultraschall zur scharfen Fokussierung amplituden- und phasengesteuert ist.

5. Verfahren nach einem der Ansprüche 1 bis 4, dadurch gekennzeichnet, daß mehrere fokussierte Ultraschallstrahlen gleichzeitig auf den subkutanen Fokus gerichtet werden.

6. Verfahren nach einem der Ansprüche 1 bis 5, dadurch gekennzeichnet, daß das Magnetfeld ein Gleichfeld ist.

7. Verfahren nach einem der Ansprüche 1 bis 7, dadurch gekennzeichnet, daß das Magnetfeld gepulst, insbesondere reihengepeist ist.

8. Verfahren nach einem der Ansprüche 1 bis 7, dadurch gekennzeichnet, daß das Magnetfeld und der Ultraschall im Fokusbereich bezüglich Phase und Frequenz abgeglichen sind.

9. Vorrichtung zur neuromagnetischen Stimulation, mit einer Magnetisierungsvorrichtung zur Beaufschlagung von zu stimulierendem Nervengewebe mit einem Magnetfeld einer magnetischen Induktion B, gekennzeichnet durch
einen Ultraschallsender (1), mit dem dem angelegten Magnetfeld ein fokussierter Ultraschallstrahl im Bereich der magnetischen Beaufschlagung derart überlagerbar ist, daß die Ultraschallwellen orthogonal zum Magnetfeld schwingen und ein subkutaner Fokus von nicht mehr als ca. 1 cm Durchmesser erzeugbar ist.

10. Vorrichtung nach Anspruch 9, dadurch gekennzeichnet, daß die Frequenz des Ultraschallsender (1) im Bereich von 0,2 bis 3 MHz, vorzugsweise im Bereich von 0,3 bis 0,5 MHz, liegt.

11. Vorrichtung nach Anspruch 9, dadurch gekennzeichnet, daß der Ultraschall gepulst, insbesondere reihengepulst ist.

12. Vorrichtung nach einem der Anssprüche 9 bis 11, gekennzeichnet durch mehrere, auf den gleichen Fokus richtbare Ultraschallsender.

# FIG. 1

# FIG. 2

EP 0 617 982 A1

# FIG. 3

7

Europäisches
Patentamt

**EUROPÄISCHER TEILRECHERCHENBERICHT,**
der nach Regel 45 des Europäischen Patentübereinkommens für das weitere Verfahren als
europäischer Recherchenbericht gilt

Nummer der Anmeldung

## EINSCHLÄGIGE DOKUMENTE

EP 94103719.4

| Kategorie | Kennzeichnung des Dokuments mit Angabe, soweit erforderlich, der maßgeblichen Teile | Betrifft Anspruch | KLASSIFIKATION DER ANMELDUNG (Int Cl.⁵) |
|---|---|---|---|
| A | US - A - 5 061 234 (CHANEY) * Zusammenfassung; Fig. 1 * | 9 | A 61 N 2/00 |
| A | US - A - 5 047 005 (CADWELL) * Zusammenfassung; Fig. 2 * | 9 | |
| A | US - A - 3 841 305 (HALLGREEN) * Zusammenfassung; Fig. 1 * | 9 | |
| A | US - A - 4 343 301 (INDECH) * Zusammenfassung; Fig. 1 * | 9 | |

RECHERCHIERTE
SACHGEBIETE (Int Cl.⁵)

A 61 N

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich
ist, auf der Grundlage einiger Patentansprüche sinnvolle Ermittlungen über den Stand der Technik
durchzuführen.

Vollständig recherchierte Patentansprüche: 9-12

Unvollständig recherchierte Patentansprüche:

Nicht recherchierte Patentansprüche: 1-8

Grund für die Beschränkung der Recherche:

Ansprüche 1-8 gemäß Art.52(4) EPÜ
nicht patentierbar; die vollständig
recherchierten Ansprüche 9-12
betreffen jedoch denselben Gegenstand.

| Recherchenort | Abschlußdatum der Recherche | Prüfer |
|---|---|---|
| WIEN | 06-07-1994 | NARDAI |